# EUROPEAN PATENT APPLICATION

(11) **EP 0 606 082 A1**
(43) Date of publication of application: **13.07.1994**
(21) Application number: 94100120.8
(22) Date of filing: 05.01.1994
(51) Int. Cl.: A61F 13/15

(54) **Curved sanitary napkin**

(30) Priority: 08.01.1993 US 2435
(71) Applicant: KIMBERLY-CLARK CORPORATION, Neenah, Wisconsin 54956-0349 (US)
(72) Inventor: McDaniel, Mary Lou, Appleton, Wisconsin 54911 (US)
(74) Representative: Diehl, Hermann, Dr. Dipl.-Phys.

(57) **Abstract**

A curved sanitary napkin (10) is disclosed which has a pair of longitudinally extending, upstanding fluid barrier walls (46,48). The sanitary napkin includes an absorbent (16) having a body-facing surface (18), a garment-facing surface (20) and a pair of longitudinally extending sides (22,24). The sides (22,24) being closest to each other approximate a central portion (26) of the absorbent (16). A liquid-impermeable baffle (28) is positioned adjacent to at least the garment-facing surface (20) of the absorbent (16) and a liquid-permeable cover (30) cooperates with the baffle (28) to enclose the absorbent (16). The cover (30) has an upper surface (32) with a pair of spaced apart longitudinal folds (34,36) integrally formed on the upper surface (32). Each of the folds (34,36) has an inside surface (38) to which is affixed an elastic member (42,44). The elastic enable the folds (36,38) to form the upstanding walls (46,48) which extend the entire length of the sanitary napkin. The upstanding walls (46,48) are aligned vertically above the longitudinal sides (22,24) of the absorbent (16) for preventing side leakage of body fluid. In addition, the elastic members (42,44)cause the sanitary napkin to acquire a cup-shaped configuration with the central portion (26) forming the deepest portion of the cup. The central portion corresponds to the perineal area of the user.

## Description

The invention relates to a curved sanitary napkin.

Sanitary napkins are designed to be worn adjacent to a person's body to absorb body fluids. Typical body fluids include menses, blood, feces, urine and other excrements. Sanitary napkins are manufactured and designed to be worn during the menstrual period to absorb large quantities of body fluids. Most sanitary napkins are susceptible to side leakage of body fluid when a large quantity of body fluid surges onto the product over an extended period of time or when the discharge is very rapid over a short period of time. In these situations, the sanitary napkin can not absorb the fluid fast enough and therefore the fluid tends to puddle on the cover and is susceptible to running off the side edges.

Various attempts to prevent or retard side leakage in sanitary napkins have been tried. Many include the use of upstanding walls located to the outside of the absorbent core. WO patent 92/07536 and European patent 0,091,412 both issued to Widlund teach such designs. Other patents which teach the use of upstanding side walls include U.S. patents 4,655,759; 4,772,282; 4,944,735 and 5,032,121. The last two of these patents improved upon the earlier sanitary napkins by combining an hour-glass shaped article with a curved or cup-shaped configuration. The hour-glass shape provided comfort during use by decreasing the amount of absorbent material present between the thighs while the curved profile allowed the absorbent article to conform to the shape of the human body. The cup shaped configuration was achieved by the use of elastic along only a portion of the longitudinal margin of the article. The limited length of the elastic allowed the central portion of the article to curve more than the ends of the article.

Still other attempts to solve side leakage are taught in U.S. patents 4,770,657; 4,701,177; 4,865,597; 4,886,513 and 5,074,856. These patents teach a sanitary napkin having a three-dimensional shape with upstanding flaps. The boat shape along with the upstanding walls made a marked improvement in preventing side leakage.

Another solution to the side leakage problem included the use of a pair of integrally formed side containment flaps. U.S. patent 4,636,207 issued to Buell and Great Britain patent 2,244,653 issued to Kobayashi et al. both teach such designs for sanitary napkins.

In the field of diapers, much attention has been directed to the use of flaps to contain fecal waste. Most teach the use of flaps located within the longitudinal side edges of the absorbent core. U.S. patents 4,704,116; 4,822,435; 4,846,823; 4,846,825; 5,032,120; 5,064,489 and 5,135,522 are representative of such designs.

Still another approach to solving the side leakage problem in sanitary napkins is described in U.S. patents 4,808,178 and 4,909,803 both issued to Aziz et al. These two patents teach a disposable absorbent article having a pair of elasticized flaps provided with leakage resistant portions. The leakage resistant portions are located above the absorbent core and inward from the longitudinal side edges.

Lastly, a further solution to the side leakage problem in sanitary napkins is taught in Japanese patent H4-152,946. In this patent, a pair of vertical walls are formed above the absorbent core and inward of the longitudinal side edges. The walls are formed only in the central portion of the article. The walls are formed from the cover material and elastic is used to secure the inside apex of each wall. The baffle is wrapped over a portion of the top surface of the absorbent and is attached to the bottom surface of the cover, below the walls. Any body fluid impinging on the central portion of the sanitary napkin would be contained between the walls until it can be absorbed by the absorbent core. The walls, which are relatively short in length, do not span the length of the sanitary napkin so that the sanitary napkin does not acquire a curved configuration.

The present invention intends to provide an absorbent article which has a curved configuration to fit the human body and which provides better protection against side leakage.

This object is solved by the curved sanitary napkin of independent claim 1. Further advantageous features, aspects and details of the napkin are evident from the dependent claims, the description and the drawings. The claims are intended to be understood as a first non-limiting approach of defining the invention in general terms.

The invention provides an absorbent article which has a curved configuration to fit the human body and which has a pair of side walls vertically aligned above the longitudinal side edges of the absorbent core.

The invention therefore provides a curved sanitary napkin with upstanding fluid barrier walls.

The invention also provides a curved sanitary napkin with a pair of upstanding fluid barrier walls to prevent side leakage of body fluid while providing improved fit and comfort. More specifically, this invention provides a curved sanitary napkin having a pair of upstanding fluid barrier walls or sidewalls aligned vertically above the longitudinal sides or side edges of the absorbent.

According to a specific aspect of the invention the sanitary napkin includes a body-facing surface, a garment-facing surface and a pair of longitudinally extending sides. The longitudinal sides are located closest to each other at the central portion of the absorbent core. A liquid-impermeable baffle is positioned adjacent to at least the garment-facing surface of the absorbent and a liquid-permeable cover cooperates with the baffle to enclose the absorbent. The cover has an upper surface with a pair of spaced apart, longitudinal folds integrally formed on the upper surface. Each of the folds has an inside surface to which is affixed an elastic member. The elastic members enable the folds to form upstanding walls which extend approximately the entire length of the sanitary napkin. The upstanding walls are aligned vertically above the longitudinal sides of the absorbent so as to prevent side leakage of body fluid. In addition, the elastic members cause the sanitary napkin to acquire a cup-shaped configuration with the central portion forming the deepest portion of the cup which corresponds to the perineal area of the user.

A general aspect of this invention is to provide a curved sanitary napkin having a pair of upstanding fluid barrier walls aligned vertically above the longitudinal sides of the absorbent to prevent side leakage. A more specific aspect of this invention is to provide a curved sanitary napkin having a pair of elasticized walls wherein the elastic also created the curved profile of the sanitary napkin.

Another aspect of this invention is to provide a curved sanitary napkin with a pair of upstanding walls vertically aligned above the longitudinal side edges of the absorbent and which contain elastic which enable the walls to remain upright while imparting a curvature to the article so that it better fits the human body.

A further aspect of this invention is to provide a curve sanitary napkin which exhibits improved side leakage protection while providing proper fit and comfort to the wearer.

Still another aspect of this invention is to provide a curved sanitary napkin which is relatively easy to manufacture.

Still further, an aspect of this invention is to provide a curved sanitary napkin which is relatively inexpensive.

Other aspects and advantages of the present invention will become more apparent to those skilled in the art in view of the following description and the accompanying drawings.

Fig. 1 is a top view of a curved sanitary napkin having an hour-glass shaped absorbent and a pair of upstanding fluid barrier walls vertically aligned above the longitudinal side edges of the absorbent.

Fig. 2 is a cross-sectional view of the curved sanitary napkin shown in Fig. 1 taken along line 2--2.

Fig. 3 is a side perspective view of the curved sanitary napkin shown in Fig. 1 with one end of the article resting on a horizontal plane to depict an angle α representing the amount of curvature in the cup-shaped sanitary napkin.

Fig. 4 is an alternative cross-sectional view of the curved sanitary napkin shown in Fig. 1 showing the use of multiple strands of elastic to retain the pair of walls in a vertical orientation relative to the longitudinal sides of the absorbent.

Fig. 5 is an alternative cross-sectional view of the curved sanitary napkin shown in Fig. 1 showing the use of adhesive to secure the cover to the absorbent and also to assist the elastic in forming the pair of upstanding walls.

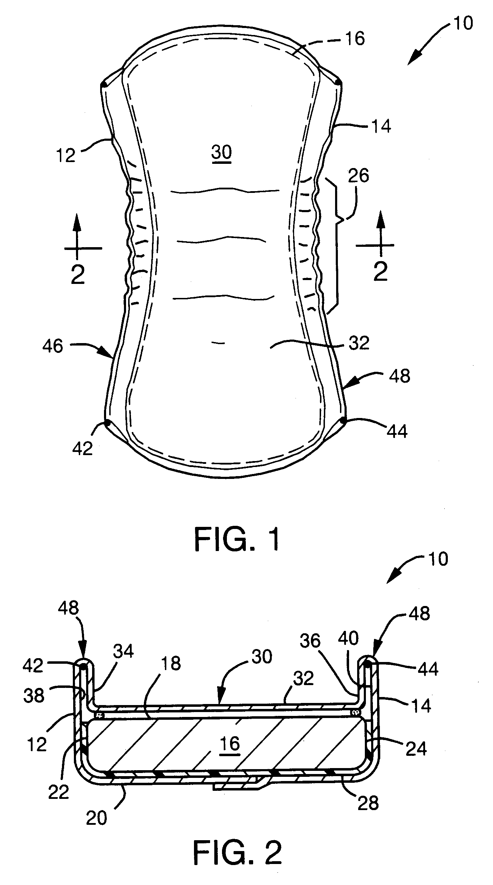

Referring to Figs. 1 and 2, a curved sanitary napkin 10 is shown which is designed to be worn adjacent to a woman's body to absorb body fluids. Typical body fluids include menses, blood, urine and other body excrements.

The sanitary napkin 10 has a pair of longitudinally extending arcuate sides 12 and 14. Located between the arcuate sides 12 and 14 is an absorbent 16 having a body-facing surface 18 and a garment-facing surface 20. The absorbent 16 also has a pair of longitudinally extending sides 22 and 24 which are aligned closest to each other approximate a central portion 26. Preferably, the absorbent 16 has an hour-glass shape, although other shapes such as a rectangle, dog bone, dumbbell, etc. can be utilized. The closeness of the sides 22 and 24 is clearly shown in Fig. 1 wherein the hour-glass shape is best depicted. In the hour-glass shape, the absorbent 16 is narrowest adjacent to the transverse axis which dissects the central portion 26.

The absorbent 16 can be a hydrophilic material formed from various types of natural or synthetic fibers including cellulose fibers, surfactant treated meltblown fibers, wood pulp fibers, regenerated cellulose or cotton fibers, or a blend of pulp and other fibers. A preferred material is coform. Coform is an air-formed blend of meltblown fibers and staple or pulp fibers. The formation of such material is disclosed in U.S. Patent 4,100,324 issued to Anderson et al. which is incorporated by reference and made a part hereof. A coform mixture of 70 percent cellulose fibers, with 30 percent polypropylene meltblown fibers, works well.

The absorbent 16 can also contain thermoplastic polymers which can be permanently deformed by the application of heat and pressure. Such materials include polypropylene, nylon, polyethylene, polyesters, etc. Typical of such materials are bonded carded webs, meltblown and spunbond fabrics.

The absorbent 16 can further contain a hydrocolloidal material, commonly referred to as a superabsorbent. The hydrocolloidal material can be a hydrogel-forming polymer composition which is water insoluble, slightly crosslinked, and partially neutralized.

The sanitary napkin 10 further includes a liquid-impermeable baffle 28 and a liquid-permeable cover 30. The liquid-impermeable baffle 28 is positioned adjacent to at least the garment-facing surface 20 of the absorbent 16 and can be constructed of a flexible plastic material, such as polypropylene, polyethylene or other suitable material. A thin polyethylene film work well. For better control in preventing body fluid from leaking out of the absorbent 16, the baffle 28 can extend upward along the sides 22 and 24 of the absorbent 16 as is shown in Fig. 2. Preferably, the ends of the baffle 28 will terminate flush with the upper or body-facing surface 18 of the absorbent 16.

The liquid-permeable cover 30 is positioned adjacent to at least the body-facing surface of the absorbent 16. However, the cover 30 can extend completely around both the absorbent 16 and the baffle 28, as is shown in Fig. 2. Alternatively, the cover 30 can cooperate with the baffle 28 to enclose the absorbent 16. The cover 30 can be a nonwoven, a spunbond or a film material which can be embossed or perforated. It is also possible to form the cover 30 from a material having a net configuration. Preferably, the cover 30 is porous to body fluids and exhibits a soft, smooth surface. The cover 30 is designed to come in contact with the body of the user and should feel comfortable while having the ability to allow human exudate in the form of menses, blood, intra-menstrual fluid, urine or other type of body fluid to readily pass through into the absorbent 16.

The cover 30 has an upper surface 32 which contacts the body of the user, and which contains a pair of spaced apart upstanding folds 34 and 36. Each of the longitudinal folds 34 and 36 has an inside surface 38 and 40, respectfully. A pair of elastic members 42 and 44 are affixed to the inside surfaces 38 and 40 of the folds 34 and 36 under tension and at least two locations. This gives the folds 34 and 36 an upstanding profile and also causes the sanitary napkin 10 to acquire a cup-shaped configuration. The elastic members 42 and 44 can be affixed to the inside surfaces 38 and 40, respectively, of the cover 30 by hot or cold melt adhesive, by ultrasonics, by a mechanical connection such as thread, or by other types of fasteners known to those skilled in the art. An adhesive attachment is preferred for it is low in cost and can be easily applied. The adhesive can be continuously or intermittently applied to the elastic members 42 and 44 depending upon one's desires and the kind of adhesive spray equipment one has available.

One way of attaching the elastic members 42 and 44 to the folds 34 and 36 is to spray an adhesive in a swirl pattern onto the inside surfaces 38 and 40 of the folds 34 and 36 and then bring the elastic members 42 and 44 into contact with the adhesive. This procedure assures that the elastic members 42 and 44 can be securely adhered to the inside surfaces 38 and 40 of the folds 34 and 36 at multiple locations without requiring a continuous coating of adhesive. Another procedure that works equally as well is to apply the adhesive to the elastic members 42 and 44 on either a continuous or intermittent basis and then bring the adhesively coated elastic members 42 and 44 into contact with the inside surfaces 38 and 40 of the folds 34 and 36.

As stated above, the pair of elastic members 42 and 44 are disposed in tension and this causes the folds 34 and 36 to form upstanding walls 46 and 48. The walls 46 and 48 extend approximately the entire length of the sanitary napkin 10, see Fig. 1. The upstanding walls 46 and 48 are arcuate along their length and are positioned so as to follow the contour of the longitudinal sides 22 and 24 of the absorbent 16. For example, when the absorbent 16 has an hour-glass profile as shown in Fig. 1, the upstanding walls 46 and 48 will track the curvilinear length of the absorbent 16.

Each of the upstanding walls 46 and 48 is aligned vertically above one of the longitudinal sides 22 and 24 of the absorbent 16. This location is important to prevent fluid flow outward toward the side edges 12 and 14 of the sanitary napkin 10. The upstanding walls 46 and 48 should be formed to the outside of the area where the body fluid would impinge upon the sanitary napkin 10. By forming the upstanding walls 46 and 48 vertically above the sides 22 and 24 of the absorbent 16, one can be assured that the body fluid, which contacts the cover 30, will have the maximum amount of time possible to be absorbed into the absorbent 16 before it contacts the upstanding walls 46 and 48. Positioning the walls 46 and 48 either inside or outside the longitudinal side edges of the absorbent 16 decreases the effectiveness of the walls 46 and 48 either by decreasing the amount of time the fluid has to be absorbed into the absorbent 16 or by allowing the fluid to flow away from the upper surface of the absorbent. When the fluid is allowed to flow off of the upper surface of the absorbent 16, there is an increased chance that body movement will distort the sanitary napkin 10 and the fluid will leak onto the wearer's skin and undergarment.

The height of the upstanding walls 46 and 48 can vary depending upon the thickness and type of sanitary napkin they are attached to. The upstanding walls 46 and 48 can have a height of from about 0.635cm to about 2.54cm (about 1/4 to about 1 inch) above the upper surface 32 of the cover 30. A height of about 0.9525 cm to about 1.27cm (about 3/8 to about 1/2 inch) serves the intended purpose of preventing side leakage of fluid from the sanitary napkin 10. A preferred height for a sanitary napkin is between about 1.27cm to about 2.54cm (about 1/2 to about 1 inch), when the central portion 26 of the sanitary napkin 10 is about 5.08cm (about 2 inches) in width.

It should be noted that the upstanding walls 46 and 48 should not be so high as to overlap one another. If this should occur, body fluid could contact the outer surface of one or both of the walls 46 and 48 and stain the wearer's undergarment. As stated above, formation of the walls 46 and 48 vertically above the longitudinal sides 22 and 24 of the absorbent 16 maximizes the width of the upper surface of the absorbent 16 which is available to receive body fluid. Accordingly, body fluid can pool on the cover 30 and flow sideways toward the upstanding walls 46 and 48 before passing down into the absorbent 16. If the upstanding walls 46 and 48 were formed closer together, less surface area of the cover 30 would be exposed to receive body fluid. This would minimize the effective fluid absorbing capacity of the sanitary napkin 10.

Furthermore, by forming the walls 46 and 48 vertically above the longitudinal sides 22 and 24 of the absorbent 16, one can determine where the upstanding walls 46 and 48 will contact the body of the user by simply varying the width of the absorbent 16 in the central portion 26. For example, by constructing an absorbent 16 with a central portion 26 which is greater than 7.62cm (3 inches) in width, one can design the sanitary napkin 10 such that the upstanding walls 46 and 48 extend into the groin of the user and form a seal with the user's skin. This body seal is advantageous in allowing the walls 46 and 48 to retard and prevent side leakage of body fluid off of the sanitary napkin 10.

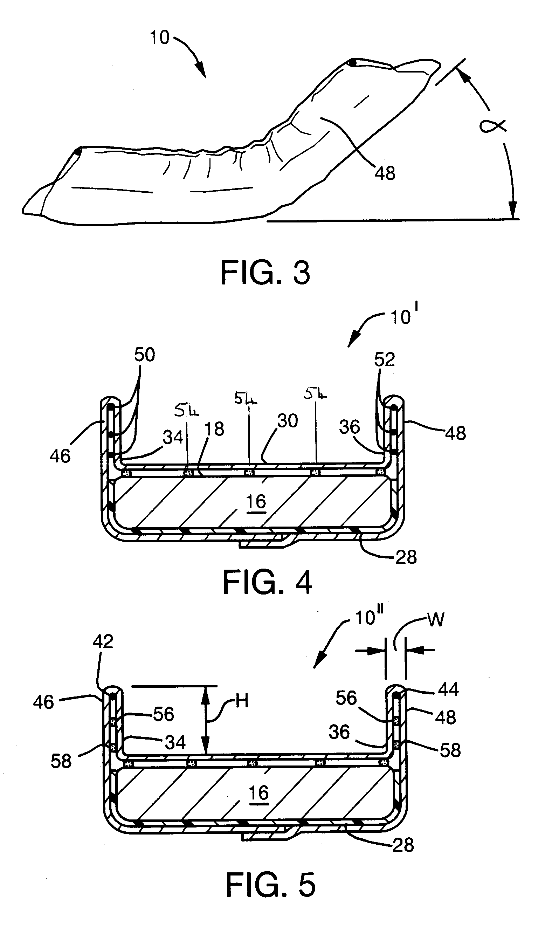

Referring to Fig. 3, the curved or cup-shaped appearance of the sanitary napkin 10 is clearly shown. This curvature is obtained by the tensile strength of the elastic members 42 and 44. The tension in the elastic members 42 and 44 as well as the method of securing the elastic members 42 and 44 to the inside surfaces 38 and 40 of the folds 34 and 36 will have an effect on the amount of curvature which will be imparted to the sanitary napkin 10. The physical size, shape and prestretch characteristics of the elastic members 42 and 44 will also affect the curvature of the sanitary napkin 10. The strength of the elastic members 42 and 44 can be adjusted such that the central portion of the sanitary napkin 10 will form the deepest portion of the cup which corresponds to the perineal area of the user. The strength of the elastic members 42 and 44 can be adjusted by varying the number of elastic strands or bands, by using elastic members having different amounts of tension or by affixing the elastic members such that their elastic characteristics are modified.

In Fig. 3, the cup-shaped configuration of the sanitary napkin 10 produces an angle α formed by the outer profile of the edge of the sanitary napkin and a plane upon which the sanitary napkin rests of between about 30° to about 90°. A preferred angle is between about 30° to about 75°.

Referring to Fig. 4, an alternative embodiment of a sanitary napkin 10' is shown in which each of the upstanding walls 46 and 48 contains two sets of three elastic members 50 and 52, respectively. The elastic members in each set 50 and 52 are vertically arranged and the tension in each strand or band can be adjusted so as to assure that the walls 46 and 48 form vertical fluid barriers. In addition, in Fig. 4, the cover 30 is bonded at several locations by adhesive 54 to the upper surface 18 of the absorbent 16. This bonding assist the folds 34 and 36 in being aligned vertically above the longitudinal sides 22 and 24 of the absorbent 16.

Referring to Fig. 5, another embodiment of a sanitary napkin 10'' is shown. In this embodiment, the upstanding walls 46 and 48 contain an elastic member 42 and 44, respectively as well as at least two strips of adhesive 56 and 58. The adhesive strips 56 and 58 can be either continuous or intermittent. The adhesive strips 56 and 58 are located below the elastic members 42 and 44. Preferably, the adhesive strips 56 and 58 are vertically aligned below the elastic members 42 and 44 so as to control the width of the folds 34 and 36. By varying the amount and location of the adhesive 56 and 58, one can create a very sturdy and flexible wall which is very thin in width.

Fig. 5 also shows that the width (W) of each of the upstanding walls 46 and 48 is less than about 20% of the height (H) of each wall. By keeping the width of the walls 46 and 48 to a minimum, one can more accurately control the vertical positioning of the walls 46 and 48 relative to the longitudinal sides 22 and 24 of the absorbent 16. The advantage of this positioning has been described above.

## Claims

1. A curved sanitary napkin (10;10';10'') having a pair of longitudinally extending arcuate sides (12,14) comprising:
a) an absorbent (16) having a body-facing surface (18) and a garment-facing surface (20), and a pair of longitudinally extending sides (22,24) which are aligned closest to each other approximate a central portion (26) of said absorbent (16);
b) a liquid-impermeable baffle (28) positioned adjacent to at least said garment-facing surface (20) of said absorbent (16);
c) a liquid-permeable cover (30) cooperating with said baffle (28) to enclose said absorbent (16), said cover (30) having an upper surface (32) and a pair of spaced apart longitudinal folds (34,36) integrally formed on said upper surface, each of said folds (34,36) having an inside surface (38); and
d) a pair of elastic members (42,44;50,52) affixed to said inside surface (38) of each of said folds (34,36) to form upstanding walls (46,48) which span approximately the entire length of said sanitary napkin, said walls (46,48) being aligned vertically above said longitudinal sides (22,24) of said absorbent (16) for preventing side leakage of body fluid, said elastic members (42,44;50,52) causing said sanitary napkin to acquire a cup-shaped configuration with said central portion (26) forming the deepest portion of said cup, said central portion corresponding to the perineal area of a user.

2. The sanitary napkin of claim 1 wherein said upstanding walls (46,48) are arcuate along their entire length.

3. The sanitary napkin of claim 1 or 2 wherein said elastic members (42,44;50,52) are disposed in tension within said upstanding walls (46,48) and said upstanding walls extend upward beyond said upper surface (32) of said cover (30) by at least 0.635cm (a 1/4 of an inch).

4. The sanitary napkin of claim 3 wherein said upstanding walls extend upward beyond said upper surface (32) of said cover (30) from 1.27cm to 2.54cm (1/2 to 1 inch).

5. The sanitary napkin of any one of the preceding claims wherein said cup-shaped configuration produces an angle α formed by the outer profile of the edge of said sanitary napkin and a plane upon which said sanitary napkin rests of between 30° to 90°.

6. The sanitary napkin of any one of the preceding claims wherein said absorbent (16) has
an hour glass shape;
said liquid permeable cover (30) surrounds both said absorbent (16) and said baffle (28); and
adhesive means (54) are provided for securing said cover (30) directly to said absorbent (16).

7. The sanitary napkin of any one of the preceding claims wherein said upstanding walls are spaced at least 5.08cm (2 inches) apart and each wall has a height of at least 1.27cm (1/2 inch) above said upper surface of said cover.

8. The sanitary napkin of any one of the preceding claims wherein each of said pair of elastic members (42,44;50,52) comprises at least one elastic band which is adhesively attached to said inside surface (38) of said folds (32,34) at two locations.

9. The sanitary napkin of claim 8 wherein said adhesive is applied to said inside surface (38) of each of said folds (34,36) in a swirl pattern and each of said elastic members (42,44;50,52) is affixed to said adhesive at several locations.

10. The sanitary napkin of any one of the preceding claims wherein each of said elastic members (42,44;50,52) has a length of at least 7.62cm (3 inches) and each of said elastic members is intermittently attached to said inside surface (38) of one of said folds (32,34).

11. The sanitary napkin of any one of the preceding claims further comprising adhesive means (56,58) for securing together portions of said upstanding walls (46,48).

12. The sanitary napkin of claim 11 wherein each fold (32,34) acquires an upstanding shape by having at least one elastic band (42,44) adhesively attached to said inside surface (38) of said fold (32,34) and at least one adhesive bond (56,58) positioned vertically below said elastic band (42,44) which is also attached to said inside surface (38) of said fold (32,34).

13. The sanitary napkin of any one of the preceding claims wherein the width (W) of each of said upstanding walls (46,48) is less than 20% of the height (H) of each of said upstanding walls.

14. The sanitary napkin of any one of the preceding claims wherein said baffle (28) extends upward around the sides (22,24) of said absorbent (16) and terminates flush with said body-facing surface (18) of said absorbent (16).
